# EUROPÄISCHE PATENTSCHRIFT

(11) **EP 2 591 735 B1**
(45) Veröffentlichungstag und Bekanntmachung des Hinweises auf die Patenterteilung: **17.05.2017**
(21) Anmeldenummer: 11189065.3
(22) Anmeldetag: 14.11.2011
(51) Int. Cl.: A61B 17/02

(54) **Verlängerung für Knochenhebel oder Wundhaken**
Extension for bone levers or retractors
Prolongation pour un levier osseux ou un écarteur de plaies

(43) Veröffentlichungstag der Anmeldung: 15.05.2013
(73) Patentinhaber: Aton AG, 3008 Bern (CH)
(72) Erfinder: Küffer, Jürg, 3008 Bern (CH)
(74) Vertreter: AMMANN PATENTANWÄLTE AG BERN

(56) Entgegenhaltungen:
- DE-U1-202005 018 651
- DE-U1-202006 005 631
- US-B1- 6 428 472

## Beschreibung

Die Erfindung betrifft eine Verlängerung für Knochenhebel oder Wundhaken, enthaltend einen Griffteil und einen Kupplungsteil zum Verbinden der Verlängerung mit dem Griffteil eines Knochenhebels oder Wundhakens, wobei der Kupplungsteil einen C-förmigen Querschnitt hat, der dazu bestimmt ist, den Griffteil eines Knochenhebels oder Wundhakens teilweise zu umschliessen.

Verlängerung für Knochenhebel oder Wundhaken, enthaltend einen Griffteil und einen Kupplungsteil zum Verbinden der Verlängerung mit dem Griffteil eines Knochenhebels oder Wundhakens.

Knochenhebel sind chirurgische Instrumente, welche zur Reposition oder zum Herausdrücken eines Knochens, gelegentlich auch als Widerlager zum Bohren, Sägen und Meisseln verwendet werden. Sie besitzen ein hakenförmiges Ende zum Erfassen des Knochens und eine Verbreiterung, die beim Hebeln als Weichteilschutz dient. Wundhaken, auch Retraktoren genannt, sind chirurgische Instrumente, mit denen der Zugang zum Operationsfeld offengehalten oder auch erst ermöglicht wird. Knochenhebel und Wundhaken bestehen meistens aus rostfreiem Stahl und weisen einen Griffteil auf, der in der Regel während der Operation von einer Hilfsperson mit der Hand festgehalten wird.

Die zunehmende Fettleibigkeit in der Bevölkerung sowie die kurze Schnittführung bei minimalinvasiven Operationstechniken führen dazu, dass bei vielen Patienten die genannten Instrumente zu kurz sind, was unter anderem dazu führt, dass sich während der Operation Hände oder doch zumindest Finger der Hilfspersonen viel zu nahe am Operationsfeld befinden und den Chirurgen bei seiner Arbeit behindern, indem sie ihm beispielsweise die Sicht auf das Operationsfeld einschränken oder versperren.

Man könnte nun einfach längere Instrumente zur Verfügung stellen, was aber insbesondere die Reinigung, Sterilisation und Lagerung der Instrumente erschweren würde. Ausserdem sind im Operationssaal sehr viele verschiedene Formen von Knochenhebeln und Wundhaken vorhanden und es wäre nachteilig, auch noch verschiedene Längen dieser Instrumente anzuschaffen und vorrätig zu haben.

Aus den Dokumenten DE202005018651U1, DE202006005631U1 und DE7813520U1 sind bereits Verlängerungen für Wundhaken bekannt. Diese Verlängerungen basieren auf Elementen, die in die im Griffbereich des Wundhakens vorhandenen Öffnungen eingreifen. Somit müssen diese Verlängerungen der Anzahl, dem Abstand, der Form und der Grösse dieser Öffnungen angepasst sein, um eine gute Verbindung zwischen dem Wundhaken und der Verlängerung zu gewährleisten. Trotzdem besteht insbesondere bei der Verlängerung nach DE7813520U1 das Risiko, dass die Verlängerung nicht starr mit dem Instrument verbunden ist und sich sogar ungewollt von diesem trennen könnte. Im Dokument US6428472B1 ist ein chirurgischer Retraktor mit einem verformbaren Griff beschrieben. Durch Verformung des Griffes kann der Benutzer die Orientierung der starren Retraktorklinge anpassen.

Ausgehend von diesem Stand der Technik liegen der Erfindung die Aufgaben zugrunde, eine Verlängerung anzugeben, die einfach und kostengünstig herstellbar ist, die für viele verschiedene Instrumente verwendbar ist und die eine sichere Verbindung mit dem jeweiligen chirurgischen Instrument gewährleistet.

Diese Aufgaben werden erfindungsgemäss dadurch gelöst, dass der Kupplungsteil gabelförmig mit zwei Gabelschenkeln ausgebildet ist.

Diese erfindungsgemässe Lösung hat insbesondere den Vorteil, dass der Griffteil eines Knochenhebels oder Wundhakens durch den Kupplungsteil der Verlängerung von aussen umschlossen wird, ohne dass in diesem Griffteil Löcher vorhanden sein müssen. Da Knochenhebel und Wundhaken in der Regel einen konischen Griffbereich haben, lässt sich durch die genannte Ausbildung des Kupplungsteils ein guter Formschluss erreichen.

Nach einer Ausführungsart besteht der C-förmige Querschnitt aus einem im Wesentlichen geraden Abschnitt und beiderseits davon je einer Umbiegung von mehr als 90 Grad. Damit kann der Kupplungsteil der Verlängerung an einem Griffbereich eines Knochenhebels oder Wundhakens anliegen, während die Umbiegungen die Ränder des Griffbereichs umfassen. Der Ausdruck Umbiegung soll dabei die Form des Erfindungsgegenstandes beschreiben, ohne diesen auf eine Herstellung durch Biegen zu beschränken.

Gemäss einer weiteren Ausführungsart nimmt die lichte Weite des C-förmigen Querschnitts gegen das freie Ende des Kupplungsteils hin ab. Durch diese Massnahme wird der Kontakt zwischen dem Kupplungsteil und einem konischen Griffteil eines Knochenhebels oder Wundhakens verbessert.

Die genannte Federwirkung kann noch verbessert werden, wenn nach einer weiteren Ausführungsart an dem vom freien Ende des Kupplungsteils abgewandten Ende einer zwischen den Gabelschenkeln vorhandenen Ausnehmung eine Erweiterung vorhanden ist.

Eine zusätzliche Ausführungsart sieht vor, dass mindestens eine der Umbiegungen durch eine nach aussen offene Ausnehmung in zwei Abschnitte geteilt ist. Die durch die Ausnehmung gebildete Materialschwächung wirkt wie ein Gelenk, um das die beiden Abschnitte relativ zueinander federnd bewegbar sind. Dadurch passt sich der Kupplungsteil noch besser der Kontur eines Griffbereichs eines Knochenhebels oder Wundhakens an.

Gemäss einer weiteren Ausführungsart ist in jedem Abschnitt eine nach innen gerichtete Erhebung angeordnet. Diese Erhebungen bilden Anlagepunkte für den Griffteil eines Knochenhebels oder Wundhakens und tragen so zu einer spielfreien Verbindung bei. Es können bevorzugt insgesamt drei oder vier Erhebungen vorhanden sein.

Nach einer anderen Ausführungsart besteht die Verlängerung aus nicht rostendem Stahl und erfüllt somit hohe Anforderungen hinsichtlich ihrer Festigkeit aber auch hinsichtlich der Hygiene.

Gemäss einer alternativen Ausführungsart besteht die Verlängerung aus Titan. Neben den vorangehend erwähnten hohen Anforderungen hinsichtlich der Festigkeit und Hygiene erfüllt eine solche Verlängerung den Wunsch eines geringen Gewichts.

Nach einer anderen Alternative kann die Verlängerung aus mit Fasern, insbesondere Kohlenstofffasern verstärktem Kunststoff bestehen. Damit erfüllt die Verlängerung mindestens annähernd die gleichen Anforderungen wie die vorangehend erwähnten Alternativen, jedoch bei geringeren Herstellungskosten.

Eine weitere Ausführungsart sieht vor, dass die Verlängerung einstückig ausgebildet ist. Diese Massnahme führt unter anderem zu einer Verbesserung der Sterilisierbarkeit.

Gemäss einer zusätzlichen Ausführungsart ist vorgesehen, dass zwischen dem Kupplungsteil und dem Griffteil ein Schieber angeordnet ist, der in Richtung der Längsachse der Hebelverlängerung über einen breiter werdenden Bereich der Hebelverlängerung schiebbar ist, um die Gabelschenkel gegeneinander zu drängen. Damit kann die Verlängerung durch Betätigen des Schiebers zusätzlich an einem Knochenhebel oder Wundhaken festgeklemmt und gesichert werden.

Um die oben erwähnte Sicherungsfunktion weiter zu verbessern, sind nach einer anderen Ausführungsart zwischen dem Schieber und der Hebelverlängerung Rastmittel angeordnet.

Schliesslich ist nach einer weiteren Ausführungsart vorgesehen, dass der Schieber einen ovalen Querschnitt hat. Dies erlaubt es, den Schieber elastisch zusammenzudrücken und damit die Rastmittel zu lockern, damit der Schieber leichter verstellbar ist.

Ein Ausführungsbeispiel der Erfindung wird nachstehend unter Bezugnahme auf die angefügten Zeichnungen beispielsweise näher beschrieben. Es zeigt
- Figur 1: den Griffteil eines bekannten Knochenhebels oder Wundhakens,
- Figur 2: eine Draufsicht auf ein Ausführungsbeispiel der erfindungsgemässen Verlängerung,
- Figur 3: eine Ansicht in Richtung des Pfeils 13 in Figur 2
- Figur 4: eine entlang der Linie 14 - 14 von Figur 3 geschnittene Ansicht in einem gegenüber Figur 3 vergrösserten Massstab und
- Figur 5: eine perspektivische Ansicht der Verlängerung.
- Figur 6: eine Draufsicht auf ein weiteres Ausführungsbeispiel der erfindungsgemässen Verlängerung,
- Figur 7: einen geschnittenen Ausschnitt aus Figur 7 und
- Figur 8: einen Querschnitt durch die Ausführungsart nach Figur 6 und 7.

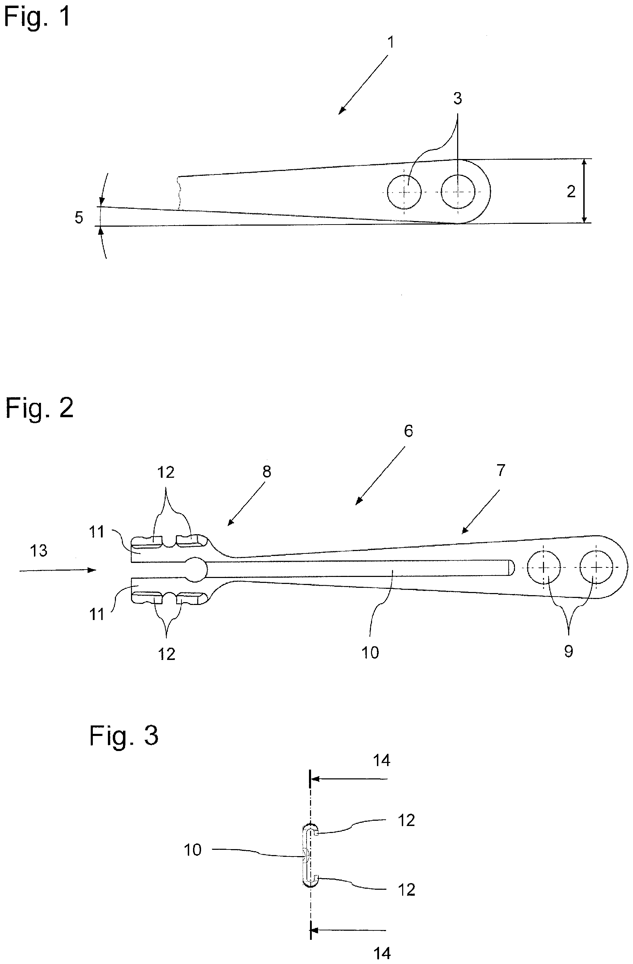

Figur 1 zeigt in einer abgebrochenen Draufsicht einen Griffteil 1 eines bekannten Knochenhebels nach Hohmann, wie er in der Norm DIN 58 845 beschrieben ist. Der Griffteil 1 hat eine Maximale Breite 2 von 28 mm mit einer Toleranz von ±2 mm und eine Dicke von ±3.6 mm mit einer Toleranz von ±0.3 mm. Im Beispiel sind im Griffteil zwei Löcher 3 vorgesehen, gemäss Normenblatt kann aber auch nur ein Loch 3 vorgesehen sein. Der Winkel 5 beträgt je nach Ausführung des Knochenhebels etwa 2° bis 3°.

Figur 2 zeigt in einer Draufsicht ein Ausführungsbeispiel einer erfindungsgemässen Verlängerung 6. Diese hat einen Griffteil 7, der prinzipiell gleich konstruiert und dimensioniert sein kann wie der Griffteil 1 eines Knochenhebels oder Wundhakens und ebenfalls mindestens ein Loch 9 aufweisen kann. Der Griffteil 1 kann aber auch anders gestaltet sein und beispielsweise einen Adapter für ein Fixiersystem aufweisen, durch welches unter Umständen bei einer Operation eine Hilfsperson eingespart werden kann. Ausserdem besitzt die Verlängerung 6 einen Kupplungsteil 8 zum Verbinden der Verlängerung 6 mit einem Knochenhebel oder Wundhaken. Eine längs verlaufende Sicke 10 kann zur Erhöhung der Biegesteifigkeit der Verlängerung 6 vorgesehen sein. Der Kupplungsteil 8 ist gabelförmig ausgebildet und hat zwei Gabelschenkel 11 mit Umbiegungen 12, deren Funktion nachstehend noch erläutert wird.

Figur 3 ist eine Ansicht in Richtung des Pfeils 13 in Figur 2. Hier sind deutlich die genannten Umbiegungen 12 zu sehen, die dem Kupplungsteil 8 eine etwa C-förmige Gestalt geben.

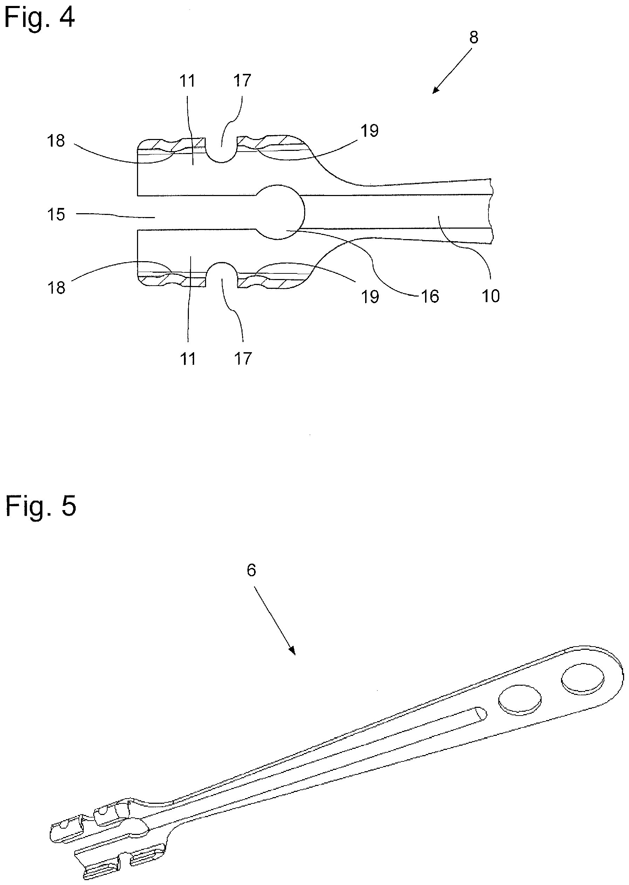

Figur 4 ist der Griffteil 8 in einem gegenüber den Figuren 2 und 3 vergrösserten Massstab dargestellt, und zwar geschnitten entlang einer durch die Pfeile 14 in Figur 3 bezeichneten Linie. Zwischen den Gabelschenkeln 11 befindet sich eine Ausnehmung 15, die an ihrem Ende eine Erweiterung 16 aufweist. In beiden Gabelschenkeln 11 ist je eine Ausnehmung 17 vorhanden, welche die betreffende Umbiegung 12 in zwei Abschnitte unterteilt. In jedem dieser Abschnitte ist im vorliegenden Beispiel eine nach innen gerichtete Erhebung 18, 19 angeordnet, die durch Verformen der Umbiegung 12 gebildet sein kann. Die lichte Weite zwischen den Erhebungen 18 ist etwas geringer als die lichte Weite zwischen den Erhebungen 19, sodass der Winkel zwischen einer die Erhebungen 18 und 19 eines Gabelschenkels 11 tangierenden Geraden und der Mittellängsachse der Verlängerung 6 mindestens etwa 3° beträgt.

In Figur 5 ist das vorangehend beschriebene Ausführungsbeispiel der erfindungsgemässen Verlängerung 6 perspektivisch dargestellt. Zum Verbinden der Verlängerung 6 mit einem Knochenhebel oder Wundhaken wird die Verlängerung über den Griffteil 1 des Knochenhebels oder Wundhakens gelegt, derart, dass der Kupplungsteil 8 an einer Stelle abseits der Löcher 3 auf dem Griffteil 1 aufliegt. Dann werden der Knochenhebel bzw. Wundhaken und die Verlängerung 6 in Richtung ihrer Längsachsen auseinandergezogen, sodass die Umbiegungen 12 den Griffteil 1 umklammern. Infolge der zuvor genannten Abstände und Winkel werden zuerst die Erhebungen 18 den Griffteil 1 seitwärts kontaktieren. Durch die Ausnehmungen 17 sind die Querschnitte der Gabelschenkel 11 geschwächt, sodass die die Erhebungen 18 enthaltenden Teile der Gabelschenkel 11 nach aussen federnd nachgeben und schliesslich auch die Ergebungen 18 seitwärts in Kontakt mit dem Griffteil 1 kommen. Durch die Ausnehmung 16 sind die Querschnitte der Gabelschenkel 11 in deren Fussbereich ebenfalls geschwächt, sodass ganzen Gabelschenkel 11 nach aussen federnd nachgeben, wenn der Knochenhebel bzw. Wundhaken und die Verlängerung 6 in Richtung ihrer Längsachsen weiter auseinandergezogen werden. Dies führt schliesslich dazu, dass alle vier Erhebungen 18, 19 an den Seitenflanken des Griffteils 1 anliegen und und eine spielfreie Verbindung zwischen dem Griffteil 1 und der Verlängerung 6 bilden.

Im vorangehend beschriebenen Beispiel ist die Verlängerung einstückig ausgebildet, was insbesondere im Hinblick auf die Sterilisation vorteilhaft ist, weil keine Fugen zwischen Einzelteilen bestehen. Die Verlängerung 6 ist vorzugsweise aus einem Edelstahl hergestellt, wie er auch für die Herstellung von Knochenhebeln und Wundhaken verwendet wird. Die Materialstärke der Verlängerung kann je nach Bedarf in einem ähnlichen Bereich liegen wie die Materialstärke im Griffbereich 1 gängiger Knochenhebel oder Wundhaken, wobei bei einer geringeren Stärke die Biegsamkeit der Verlängerung erhöht ist, was erwünscht sein kann, weil dadurch das Gewebe des Patienten rund um das Operationsfeld geschont wird. Die Verlängerung muss nicht wie dargestellt gerade ausgebildet sein, sondern kann auch eine Biegung aufweisen. Nach Bedarf kann die Verlängerung wie erwähnt eine längsverlaufende Sicke 10 aufweisen, um die Steifigkeit zu erhöhen. Die Verlängerung 6 kann durch spanlose Formgebung hergestellt werden, beispielsweise durch Umbiegen der betreffenden Bereiche 12 aus einem flachen Material. Es ist aber auch möglich, die Verlängerung 6 durch spanabhebende Bearbeitung herzustellen.

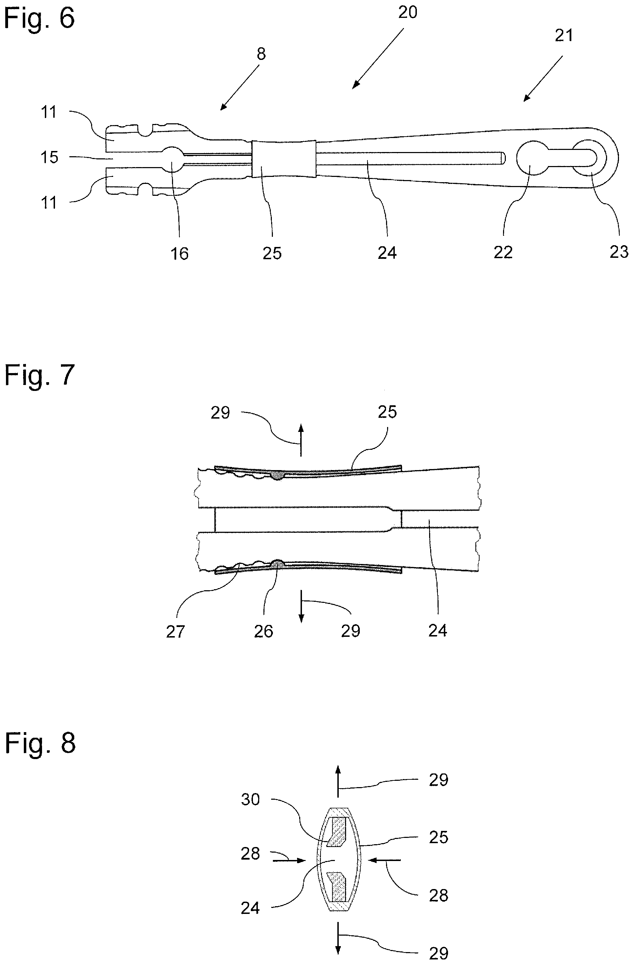

Die Figuren 6 bis 8 zeigen eine weitere Ausführungsart der erfindungsgemässen Hebelverlängerung. Figur 8 zeigt in einer Darstellung entsprechend Figur 2 eine Draufsicht auf diese Ausführungsart der Hebelverlängerung, die in diesem Fall mit der Bezugszahl 20 bezeichnet ist. Auch in dieser Ausführungsart hat die Hebelverlängerung 20 einen Kupplungsteil 8, der prinzipiell gleich ausgebildet ist, wie der Kupplungsteil 8 gemäss Figur 2, nämlich mit zwei Gabelschenkeln 11 und einer dazwischen angeordneten Ausnehmung 15. Im Unterschied zur Ausführungsart nach Figur 2 hat die Ausführungsart nach den Figuren 6 bis 8 einen entlang der Längsachse der Hebelverlängerung 20 verlaufenden Schlitz 24, der bei der Erweiterung 16 beginnt und im Bereich des Griffteils 21 endet. Es ist offensichtlich, dass durch diesen Schlitz 24 die beiden Gabelschenkel 11 wesentlich leichter elastisch gegeneinander und voneinander bewegbar sind, als bei der Ausführungsart nach Figur 2. Ein Schieber 25, dessen Funktion nachstehend unter Bezugnahme auf die Figuren 7 und 8 näher erläutert wird, hält die durch den Schlitz 24 getrennten Bereiche der Hebelverlängerung 20 zusammen. Abweichend von der Gestaltung des Griffteils 7 von Figur 2 enthält der Griffteil 21 eine schlüssellochartige Öffnung 22, an deren schmalerem Ende eine Vertiefung 23 angeordnet ist. Damit ist dieser Griffteil 21 besonders gut geeignet, an einem Fixiersystem angekuppelt zu werden, mit dem eine Hilfsperson zum Halten des Knochenhebels oder Wundhakens eingespart werden kann.

Figur 7 zeigt den Schieber 25 in einer geschnittenen Ansicht und in einem gegenüber Figur 6 vergrösserten Massstab. Links in Figur 7 ist zu erkennen, dass die Breite der Hebelverlängerung in Richtung auf den Kupplungsteil zunimmt und dass in den Seitenkanten der Hebelverlängerung Ausnehmungen 27 angeordnet sind. Der Schieber 25 hat an seiner Innenseite Nocken 26, die in diese Ausnehmungen 27 eingreifen und damit eine Rastrierung bilden. Durch Bewegen des Schiebers 25 in Richtung auf den Kupplungsteil werden die Gabelschenkel aufeinander zu bewegt beziehungsweise mit zunehmender Kraft gegen einen zwischen den Gabelschenkeln 11 aufgenommenen Knochenhebel oder Wundhaken gedrückt. Damit ist die verlängerung 20 am Wunhaken oder Knochenhebel zusätzlich gesichert und kann grössere Kräfte übertragen. Zum leichteren Verschieben des Schiebers 25 können Massnahmen vorgesehen sein, die es ermöglichen, den Schieber in Richtung der Pfeile 29 zu dehnen und die nachstehend unter Bezugnahme auf die Figur 8 beschrieben werden.

Figur 8 zeigt einen Schnitt durch die Hebelverlängerung 20 im Bereich des Schiebers 25, wobei ersichtlich ist, dass der Schieber 25 einen etwa ovalen Querschnitt hat und so gestaltet ist, dass er nicht auf den breiten Seiten der Hebelverlängerung 20 aufliegt. Wenn nun dieser Schieber 25 auf seinen breiten Seiten in Richtung der Pfeile 28 zusammengedrückt wird, bewegen sich die schmalen Seiten des Schiebers 25 in Richtung der Pfeile 29 auseinander, so dass die Nocken 26 aus den Ausnehmungen 27 gehoben werden und sich der Schieber 25 leichter in Richtung der Längsachse der Hebelverlängerung 20 verschieben lässt. Die Figur 8 zeigt ausserdem, dass auch dieser Hebelverlängerung 20 eine erhöhte Biegesteifigkeit verliehen werden kann, indem die Randbereiche 30 des Schlitzes 24 etwas abgebogen sind.

### Bezugszeichenliste

- 1: Griffteil (Stand der Technik)
- 2: Breite
- 3: Löcher
- 4 5: Winkel
- 6: Hebelverlängerung
- 7: Griffteil
- 8: Kupplungsteil
- 9: Löcher
- 10: Sicke
- 11: Gabelschenkel
- 12: Umbiegungen
- 13: Pfeil
- 14: Pfeil
- 15: Ausnehmung
- 16: Erweiterung
- 17: Ausnehmung
- 18: Erhebung
- 19: Erhebung
- 20: Hebelverlängerung
- 21: Griffteil
- 22: Öffnung
- 23: Vertiefung
- 24: Schlitz
- 25: Schieber
- 26: Nocken
- 27: Ausnehmung
- 28: Pfeile
- 29: Pfeile
- 30: Randbereich von 23

## Patentansprüche

1. Verlängerung (6; 20) für Knochenhebel oder Wundhaken, enthaltend einen Griffteil (7; 21) und einen Kupplungsteil (8) zum Verbinden der Verlängerung mit dem Griffteil (1) eines Knochenhebels oder Wundhakens, wobei der Kupplungsteil (8) einen C-förmigen Querschnitt hat, der dazu bestimmt ist, den Griffteil (1) eines Knochenhebels oder Wundhakens teilweise zu umschliessen, **dadurch gekennzeichnet, dass** der Kupplungsteil (8) gabelförmig mit zwei Gabelschenkeln (11) ausgebildet ist.

2. Verlängerung (6; 20) nach Anspruch 1, **dadurch gekennzeichnet, dass** der C-förmige Querschnitt aus einem im Wesentlichen geraden Abschnitt und beiderseits davon je einer Umbiegung (12) von mehr als 90 Grad besteht.

3. Verlängerung (6; 20) nach einem der vorangehenden Ansprüche, **dadurch gekennzeichnet, dass** die lichte Weite des C-förmigen Querschnitts gegen das freie Ende des Kupplungsteil (8) hin abnimmt.

4. Verlängerung (6; 20) nach einem der vorangehenden Ansprüche, **dadurch gekennzeichnet, dass** an dem vom freien Ende des Kupplungsteils (8) abgewandten Ende einer zwischen den Gabelschenkeln (11) vorhandenen Ausnehmung (15) eine Erweiterung (16) vorhanden ist.

5. Verlängerung (6; 20) nach einem der Ansprüche 2 bis 4, **dadurch gekennzeichnet, dass** mindestens eine der Umbiegungen (12) durch eine nach aussen offene Ausnehmung (17) in zwei Abschnitte geteilt ist.

6. Verlängerung (6; 20) nach Anspruch 5, **dadurch gekennzeichnet, dass** in jedem Abschnitt eine nach innen gerichtete Erhebung (18, 19) angeordnet ist.

7. Verlängerung (6; 20) nach einem der vorangehenden Ansprüche, **dadurch gekennzeichnet, dass** sie aus nicht rostendem Stahl besteht.

8. Verlängerung (6; 20) nach einem der Ansprüche 1 bis 6, **dadurch gekennzeichnet, dass** sie aus Titan besteht.

9. Verlängerung (6; 20) nach einem der Ansprüche 1 bis 6, **dadurch gekennzeichnet, dass** sie aus mit Fasern, insbesondere Kohlenstofffasern verstärktem Kunststoff besteht.

10. Verlängerung (6) nach einem der vorangehenden Ansprüche, **dadurch gekennzeichnet, dass** sie einstückig ausgebildet ist.

11. Verlängerung (20) nach einem der Ansprüche 1 bis 9, **dadurch gekennzeichnet, dass** zwischen dem Kupplungsteil (8) und dem Griffteil (7; 21) ein Schieber (25) angeordnet ist, der in Richtung der Längsachse der Hebelverlängerung (20) über einen breiter werdenden Bereich der Hebelverlängerung (20) schiebbar ist, um die Gabelschenkel (11) gegeneinander zu drängen.

12. Hebelverlängerung nach Anspruch 11, **dadurch gekennzeichnet, dass** zwischen dem Schieber und der Hebelverlängerung Rastmittel (27, 28) angeordnet sind.

13. Hebelverlängerung nach einem der Ansprüche 11 bis 12, **dadurch gekennzeichnet, dass** der Schieber (25) einen ovalen Querschnitt hat.

## Claims

1. Extension (6; 20) for bone levers or retractors, comprising a handle portion (7; 21) and a coupling portion (8) for connecting the extension to the handle portion (1) of a bone lever or retractor, the coupling part (8) having a C-shaped cross-section that is intended to partly enclose the handle portion (1) of a bone lever or retractor, **characterised in that** the coupling portion (8) is forkshaped and has two fork shanks.

2. Extension (6; 20) according to claim 1, **characterised in that** the C-shaped cross-section consists of a substantially straight section and of bends (12) of more than 90 degrees on both sides thereof.

3. Extension (6; 20) according to one of the preceding claims, **characterised in that** the inner width of the C-shaped cross-section decreases towards the free end of the coupling portion (8).

4. Extension (6; 20) according to one of the preceding claims, **characterised in that** an enlargement (16) is provided at the end opposite the free end of the coupling portion (8) of a recess (15) between the fork legs (11).

5. Extension (6; 20) according to one of claims 2 to 4, **characterised in that** at least one of the bends (12) is divided into two sections by an outwardly open recess (17).

6. Extension (6; 20) according to claim 5, **characterised in that** in each section there is arranged an inwardly facing raised portion (18, 19).

7. Extension (6; 20) according to one of the preceding claims, **characterised in that** it is made of stainless steel.

8. Extension (6; 20) according to one of claims 1 to 6, **characterised in that** it is made of titanium.

9. Extension (6; 20) according to one of claims 1 to 6, **characterised in that** it is made of fibre-reinforced, more particularly carbon fibre-reinforced plastic material.

10. Extension (6; 20) according to one of the preceding claims, **characterised in that** it is made of one piece.

11. Extension (6; 20) according to one of claims 1 to 9, **characterised in that** between the coupling portion (8) and the handle portion (7; 21) there is arranged a slider (25) that is slidable over an widening section of the lever extension (20) in the direction of the longitudinal axis of the lever extension (20) in order to press the fork legs (11) against each other.

12. Lever extension according to claim 11, **characterised in that** snap means (27, 28) are arranged between the slider and the lever extension.

13. Lever extension (6; 11) according to one of claims 11 to 12, **characterised in that** the slider (25) has an oval cross-section.

## Revendications

1. Rallonge (6; 20) pour leviers à os ou écarteurs, comprenant une partie manche (7; 21) et une partie d'accouplement (8) permettant de relier la rallonge à la partie manche (1) d'un levier à os ou d'un écarteur, la partie d'accouplement (8) présentant une section transversale en forme de C qui est destinée à entourer partiellement la partie manche (1) d'un levier à os ou d'un écarteur, **caractérisée en ce que** la partie d'accouplement (8) est fourchue avec deux branches de fourche.

2. Rallonge (6; 20) selon la revendication 1, **caractérisée en ce que** la section en forme de C est composée d'une partie substantiellement droite et de courbures (12) respectives des deux côtés de celle-ci.

3. Rallonge (6; 20) selon l'une des revendications précédentes, **caractérisée en ce que** la largeur intérieure de la section en forme de C diminue vers l'extrémité libre de la partie d'accouplement (8).

4. Rallonge (6; 20) selon l'une des revendications précédentes, **caractérisée en ce qu'**un élargissement (16) est pourvu à l'extrémité opposée à l'extrémité libre de la partie d'accouplement (8) d'une entaille (15) pourvue entre les branches de fourche (11).

5. Rallonge (6; 20) selon l'une des revendications 2 à 4, **caractérisée en ce qu'**au moins une des courbures (12) est divisée en deux parties par une entaille (17) ouverte vers l'extérieur.

6. Rallonge (6; 20) selon la revendication 5, **caractérisée en ce que** dans chacune desdites parties est agencée une saillie (18, 19).

7. Rallonge (6; 20) selon l'une des revendications précédentes, **caractérisée en ce qu'**elle est en acier inoxydable.

8. Rallonge (6; 20) selon l'une des revendications 1 à 6, **caractérisée en ce qu'**elle est en titane.

9. Rallonge (6; 20) selon l'une des revendications 1 à 6, **caractérisée en ce qu'**elle est en matériau synthétique renforcé de fibres, plus particulièrement de fibres de carbone.

10. Rallonge (6; 20) selon l'une des revendications précédentes, **caractérisée en ce qu'**elle est faite d'une seule pièce.

11. Rallonge (6; 20) selon l'une des revendications 1 à 9, **caractérisée en ce qu'**entre la partie d'accouplement (8) et la partie manche (7; 21) est agencé un coulisseau déplaçable dans la direction de l'axe longitudinal de la rallonge de levier (20) sur une partie de la rallonge de levier (20) dont la largeur augmente, afin de forcer les branches de fourche (11) l'une contre l'autre.

12. Rallonge de levier selon la revendication 11, **caractérisée en ce qu'**entre le coulisseau et la rallonge de levier sont agencés des moyens d'encliquetage (27, 28).

13. Rallonge de levier selon l'une des revendications 11 à 12, **caractérisée en ce que** le coulisseau (25) présente une section ovale.
